# EUROPEAN PATENT SPECIFICATION

(11) **EP 2 603 180 B1**
(45) Date of publication and mention of the grant of the patent: **08.03.2017**
(21) Application number: 11757423.6
(22) Date of filing: 09.08.2011
(51) Int. Cl.: A61F 5/00

(54) **ADAPTER FOR STOMACH DEVICES**
ADAPTER FÜR MAGENVORRICHTUNGEN
ADAPTATEUR POUR DISPOSITIFS GASTRIQUES

(30) Priority: 02.05.2011 US 201161472205 P; 01.03.2011 GB 201103467; 10.08.2010 US 372257 P
(43) Date of publication of application: 19.06.2013
(73) Proprietor: Yissum Research Development Company of the Hebrew University of Jerusalem Ltd., 97390 Jerusalem (IL)
(72) Inventor: WEISS, Ram, 34485 Haifa (IL); WEISS, Menachem, P., 34485 Haifa (IL)
(74) Representative: Pearl Cohen Zedek Latzer Baratz UK LLP
(86) International application number: PCT/IB2011/053550
(87) International publication number: WO 2012/020375

(56) References cited:
- WO-A1-2004/064685
- WO-A2-2008/013862
- US-A1- 2005 246 037
- US-A1- 2005 267 499
- US-A1- 2006 276 812

## Description

### BACKGROUND

### 1. TECHNICAL FIELD

The present invention relates to the field of gastric medical devices, and more particularly, to an endoscopic device connectable to the tissues inside the stomach.

### 2. DISCUSSION OF RELATED ART

Obesity is reaching epidemic proportions in the Western as well as the developing world. Along with the increase in the prevalence of simple obesity, there is a growing population of morbidly obese individuals (BMI > 40 kg/m²) as well as super obese ones (BMI > 50 kg/m²). As obesity carries increased risk of mortality and a broad spectrum of related co-morbidity, treatment approaches aimed at addressing this problem are needed. The conservative and most successful approach is of lifestyle modifications that include dietary changes and increased physical activity. The problem with this approach is the low compliance achieved and the limited benefit it provides for those with morbid and super obesity. There are several present and emerging pharmacological agents aimed at treating obesity yet their benefit seems to provide a modest and unsustainable weight loss, along with unpleasant side effects, and is diminished upon discontinuation of the drug.

The lack of success of conservative and pharmacological approaches to treat obesity lead to the emergence of bariatric surgery as the most effective and sustainable treatment option. Moreover, some bariatric surgical procedures result in hormonal changes that lead to improvement of conditions such as altered glucose metabolism and thus these procedures are also called "metabolic surgery".

Bariatric procedures have two general mechanisms of action - restrictive and malapsorptive. The vast majority is performed using a laparoscopic approach and a laparotomy is rarely necessary. The simplest restrictive procedure is gastric banding in which an adjustable elastic band is used to create a small gastric pouch. The radius of the band can be changed using its subcutaneous bladder. Another procedure that is gaining popularity is the sleeve gastrectomy where a large portion of the stomach is resected leaving a narrow "sleeve" with limited volume. This procedure is claimed to have "metabolic" effects via reduction of ghrelin. The classic bariatric procedure is the Roux-en-Y gastric bypass (RYGB). In this procedure a small gastric pouch is created and anastomosed to a distal part of the small bowel while the stomach and duodenum are anastomosed distally. This leaves the proximal part of the small bowel without pancreatic enzymes and bile salts and reduces the area of absorption. Thus, the RYGB combines a restrictive and malapbsorptive component.

All bariatric procedures cause a greater weight loss than conservative approaches yet carry small but significant operative and peri-operative risks. This has lead to attempts to perform these procedures or imitate their effects via less invasive approaches. The simple approach to the gastro-intestinal tract is via the oral route, thus attempts at designing bariatric procedures that are performed using endoscopic machinery are actively pursued. The simplest one is the gastric balloon or other volume occupying devices that limit gastric capacity. These devices have been demonstrated to have a small short term effect on weight yet have significant side effects that have limited their use in clinical practice. Other approaches have attempted to suture the gastric wall using an endoscope thus reducing its volume and thus imitating the gastric sleeve procedure. Yet another approach contemplated a variable outlet that can be changed manually using a laparoscopic or endoscopic mechanism similar to the classic gastric band. Another attempt has been made to combine the restrictive and malabsorptive approach by connecting a flexible tube to the vicinity of the gastro-esophageal junction and leading it via the pylorus through the duodenum for a variable length. This approach creates a limited gastric pouch and adds a bypass component.

GERD is a common condition afflicting millions of adults and children that results from an anatomical or regulatory derangement of the gastro-esophageal sphincter that allows reflux of acid gastric contents into the esophagus. This condition is usually treated by pharmacological means and in severe case -by surgical means. There is currently no useful intra-gastric device to address this medical problem.

U.S. Patent No. 7037344 discloses an artificial stoma device, a gastric sleeve device, an intestinal sleeve device and a combined gastrointestinal sleeve device. The following documents disclose various gastric pouches: U.S. Patent Nos. 4403604, 6981978, 7037344, and 7267694, U.S. Patent Publication Nos. 20040122453, 20050267499, 2005240279, 20090012541, 2009093839, and 20100114130. A medical device according to the preamble of claim 1 is disclosed in US-A-2005/0267499.

### BRIEF SUMMARY

The invention is defined in independent claim 1. Preferred embodiments are defined in dependent claims 2-12.

Embodiments of the present invention provide a medical device comprising: an inert tubular adapter comprising a proximal rim arranged to be connectable to an inner lining of the stomach to encircle the esophageal sphincter, and a treatment element affixed to the inert tubular adapter; characterized in that the device has an active state in which the treatment element is operative to constrict the passage of food to the and an inactive state in which the treatment element is not operative, the treatment element having a delayed activation mechanism, the tubular adapter and the treatment element are arranged to be endoscopically inserted into the stomach by a single insertion procedure, with the treatment element in the inactive state, and the delayed activation mechanism is arranged to be mechanically or chemically self-activated or activated externally after a specified period following insertion.

These, additional, and/or other aspects and/or advantages of the present invention are: set forth in the detailed description which follows; possibly inferable from the detailed description; and/or learnable by practice of the present invention.

### BRIEF DESCRIPTION OF THE DRAWINGS

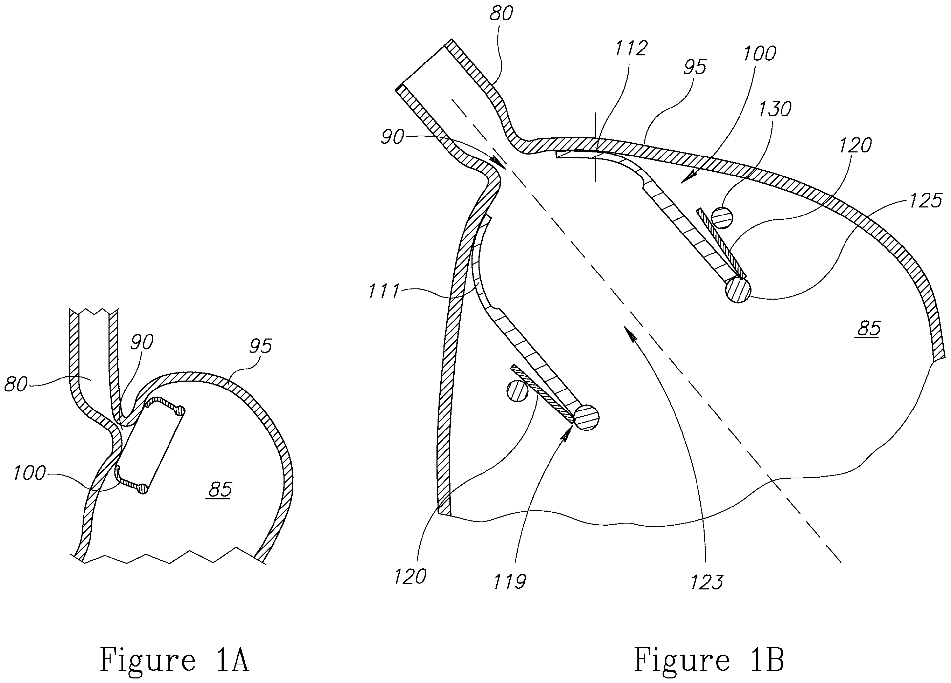

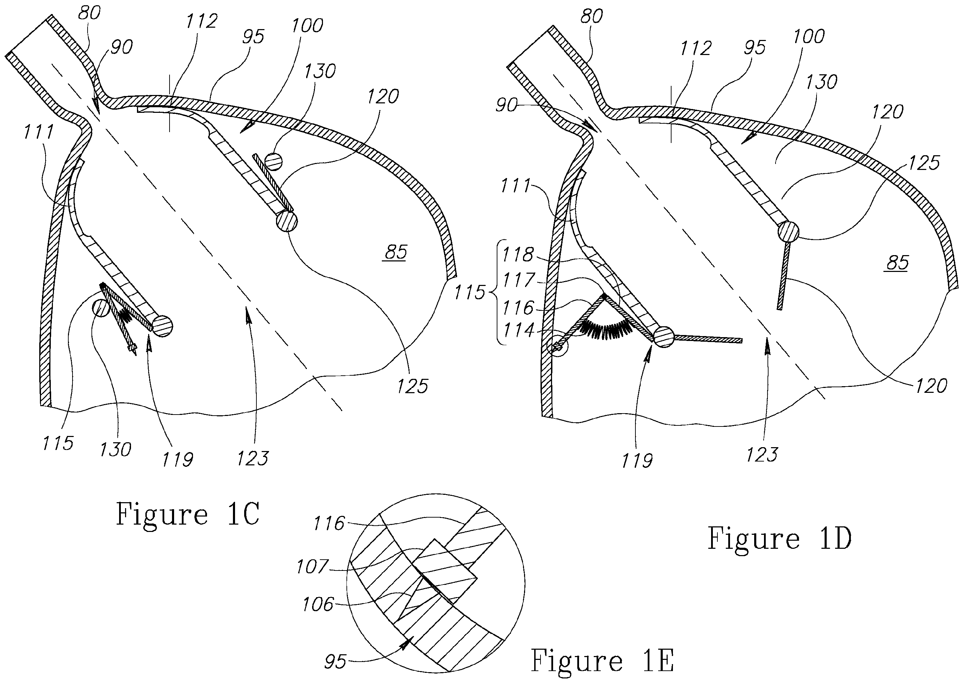

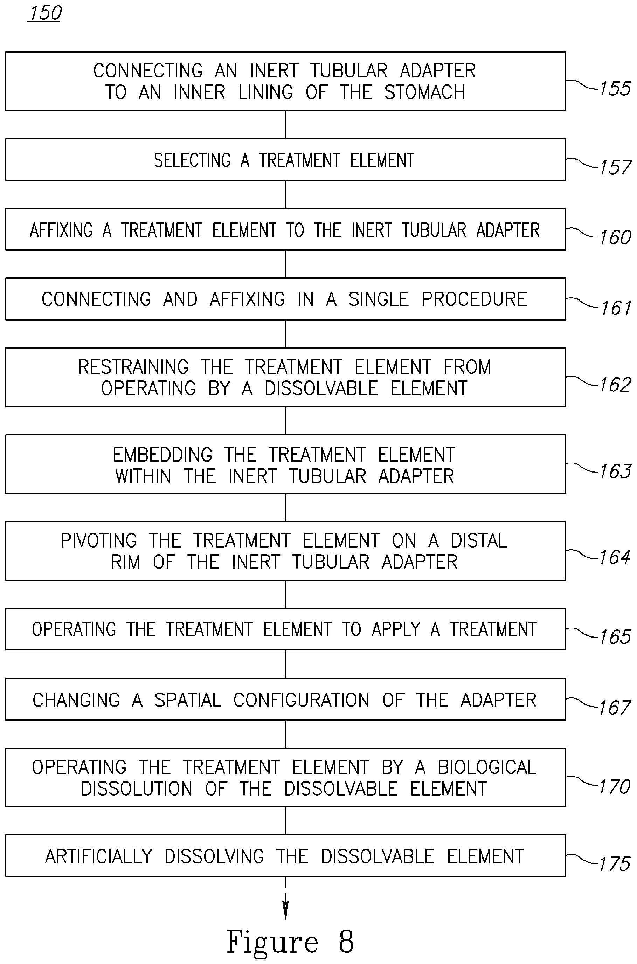

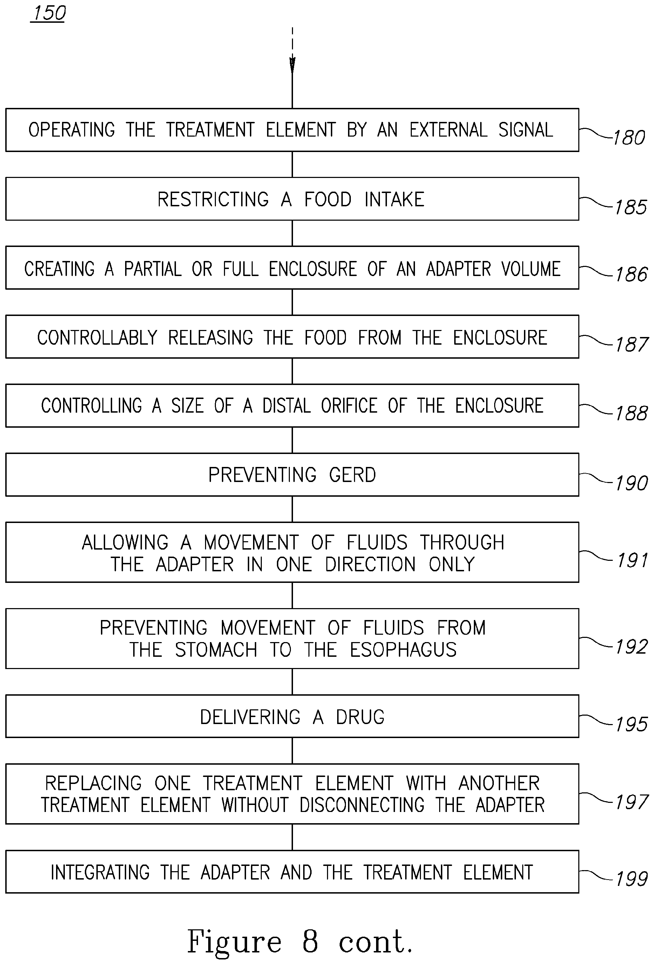

The present invention will be more readily understood from the detailed description of embodiments thereof made in conjunction with the accompanying drawings of which: Figures 1 to 7 are high level schematic illustrations of a medical device, according to some embodiments of the invention; and Figure 8 is a high level flowchart illustrating a gastric treatment method, according to some embodiments of the invention.

### DETAILED DESCRIPTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

For a better understanding of the invention, the term "intake limiting pouch" in the present disclosure is defined in a non-limiting manner as an artificial mechanical structure (unlike common gastric pouches made at least in part from the stomach lining) connected within the stomach and encircling the esophageal sphincter, that receives the intake of food and liquids coming in through the esophageal sphincter, and at least partially holds the intake for a specified period of time, to limit the amount of possible intake in that time. The intake limiting pouch may hold and release the intake, hold and release a part of the intake, or create a mechanical barrier that postpones the transition of the intake from the esophagus to the stomach.

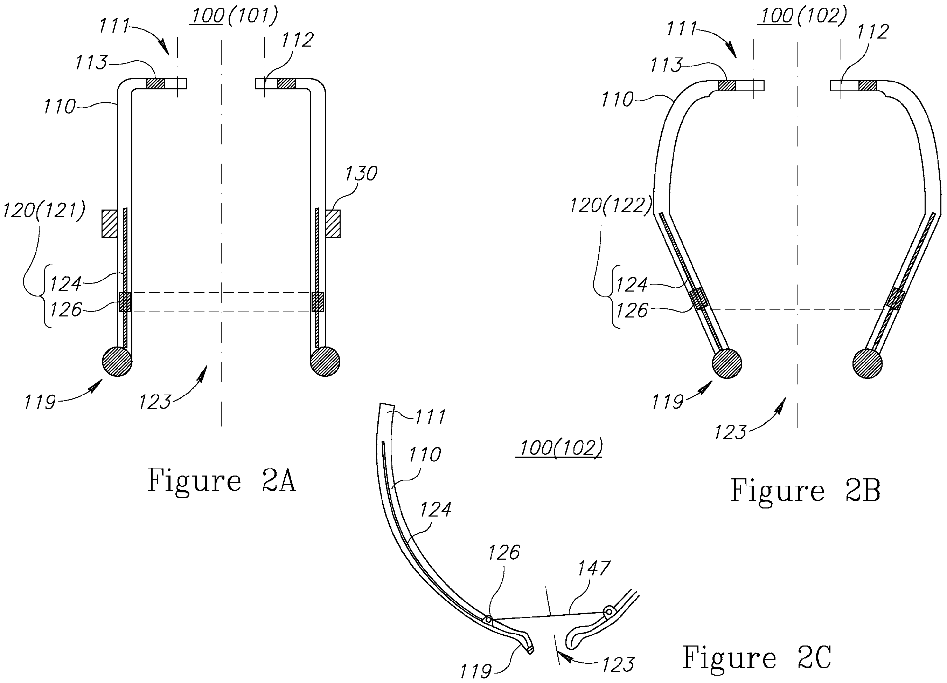

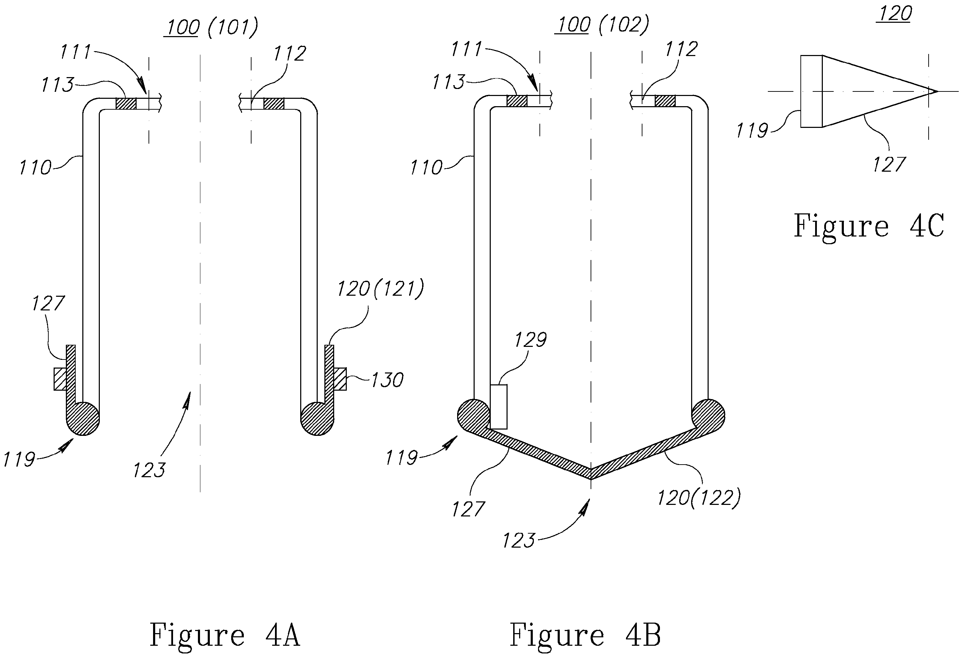

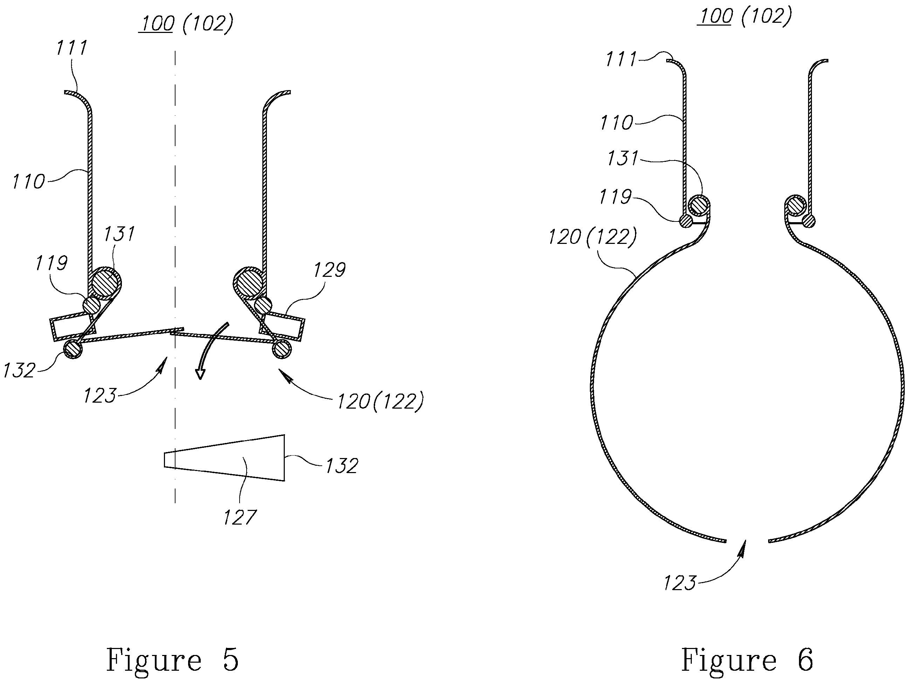

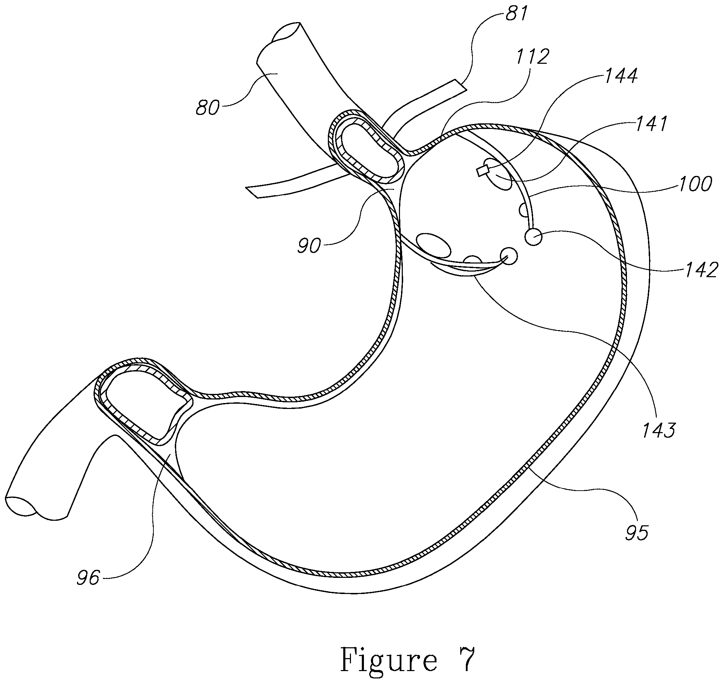

**Figures 1** to **6** are high level schematic illustrations of a medical device **100,** according to some embodiments of the invention. **Figures 1A** to **1E** are cross sections of device **100** within the stomach **85,** **Figures 2** to **6** are cross sections of various configurations of device **100** in a two part configuration, and **Figure 7** illustrates device **100** as an intake limiting pouch in a single part configuration. **Figures 2B, 2C****,** **3B****,** **6** and **7** illustrate device **100** operating as an intake limiting pouch, while **Figures 4B** and **5** illustrate device **100** operating as an anti GERD one way valve.

Medical device **100** comprises an inert tubular adapter **110** that is connected to the inner lining **95** of stomach **85,** and a treatment element **120** (or parts thereof) that is affixable to inert tubular adapter **110.** Treatment element **120** (or parts thereof) has an operative state **122** in which it is arranged to apply a treatment such as turning device **100** to an intake limiting pouch or to an anti-GERD one way valve, or to deliver a drug.

Medical device **100** has an inactive state **101,** in which treatment element **120** is not operative or is not affixed to adapter **110,** and an activated state **102,** in which treatment element **120** is operative (**122**).

Tubular adapter **110** may have a diameter that is larger than the diameter of sphincter **90** in its maximal opening size, to allow some mechanical freedom to both device **100** and stomach lining **95.**

Device **100** uses stomach lining **95** only as an anchoring area, and does not utilize lining **95** build the pouch or any parts of device **100.**

Medical device **100** is arranged to be activated only a specified period after the connection of tubular adapter **110** to inner lining **95** of stomach **85.** The specified period is equal to or longer than the healing period of the connection itself, to separate temporally the connection of tubular adapter **110** and the application of the treatment.

For example, medical device **100** may be assembled prior to its insertion to stomach **85,** inserted and attached endoscopically to inner lining **95,** and have a delayed activation mechanism. Alternatively, only adapter **110** may be inserted and attached endoscopically to inner lining **95,** and after the specified period, only treatment element **120** may be inserted endoscopically and attached to adapter **110.** Treatment element **120** may be operative (**122**) directly after its attachment to adapter **110,** or activation of device **100** may be further delayed by a holding element **130,** as described below. Device **100** may be self activated by a mechanical or chemical delayed release mechanism, or activated externally by a signal of any kind. Additionally, treatment element **120** may be replaced endoscopically after or even before a first use upon corresponding indications.

In embodiments, inert tubular adapter **100** may be connected to stomach **85** at an occurring opportunity and stay inert and inactivated for a long period within stomach **85.** Treatment element **120** may be operated (**122**) or connected to adapter **110** at any later occasion.

In contrast to prior art methods and devices, medical device **100** temporally separates the attachment phase from the beginning of treatment, and ensures thereby both proper healing of attachment area **112** before the commencement of the treatment as well as the safety of the associated force application.

During the healing period the patient is not limited in food consumption as the inner passage of the adapter is larger than esophageal sphincter **90.** In this way, force application on connection area **112** (sutures or other means of connection) is avoided, and good healing of attachment area **112** is achieved. Hence, in contrast to prior art methods and devices which require supervision and restrictions to food consumption during the healing period, patients treated in the proposed procedures are not limited in regards to food consumption during the healing period. Furthermore, the treating physicians have a better control on the treatment, as device **100** allows a better control on the commencement of the treatment, and also allows temporary or permanent cessation of the treatment by changing the state of treatment element **120** to inoperative, or removing treatment element **120** altogether, without wounding or damaging stomach **85,** as adapter **110** may be left connected in stomach **85.** Exchanging treatment element **120** may also be carried out endoscopically, without damaging the connection of adapter **110** to stomach **85.** Any or both adapter **110** and treatment element **120** may be removed from stomach **85** by a single procedure.

Inert tubular adapter **110** comprises a proximal rim **111** and a distal rim **119.** Proximal rim **111** is arranged to be connectable to inner lining **95** of stomach **85,** to encircle the esophageal sphincter **90,** such that the fluid connection of stomach **85** with esophagus **80** is carried out via device **100.**

Proximal rim **111** may be arranged to enable its connection (**112**) to inner lining **95** of stomach **85** by suturing, gluing, clipping, stapling, and riveting. Riveting may comprise using blind rivets with expanding large caps on the opposite side of the insertion side. Riveting may utilize non-blind rivets. The rivets may be connected to each other on the serosal side of the tissue.

The whole or part of medical device **100** may have an inactive state **101** and an activated state **102,** for example a deformation of tubular adapter **110** may participate in the activation of device **100.**

Treatment element **120** is affixable to distal rim **119** and has operative state **122** arranged to apply a treatment. Operative state **122** may comprise a mechanical deformation, a position change, a change in chemical character or any other change that affects the stomach or the food passing through device **100.**

Tubular adapter **110** may be arranged to receive treatment element **120** after the specified healing period has passed. For example (**Figures 1B****,** **3A** and **4A**), distal rim **119** may comprise a flange **125** arranged to engage and affix treatment element **120** that is inserted after the healing period. The insertion of treatment element **120** does not involve any tissue injury and its connection to the already established tubular adapter **110** is purely mechanical. This insertion stage may also be use to control the healing and ensure the safe onset of the treatment.

In embodiments, treatment element **120** may be affixed to the adapter walls or be embedded within the walls (see below, **Figures 1B****,** **2A,** and **2B**). In inactive state **101** of device **100,** treatment element **120** may be at least partially inserted within a body of inert tubular adapter **100,** and in active state **102** of device **120,** treatment element **120** may either stay embedded within adapter **110** causing it to mechanically deform, or may be at least partially released from the adapter body to assume operative state **122.**

Tubular adapter **110** and treatment element **120** may be arranged to be endoscopically inserted into stomach **85** by a single insertion procedure, with treatment element **120** in an inoperative state **121.** A transition of treatment element **120** from inoperative state **121** to operative state **122** may be carried out upon a specified condition that takes place after the specified healing period.

Medical device **100** may comprise at least one holding element **130** such as dissolvable elements arranged to change the state of treatment element **120** from inoperative **121** to operative **122,** or the state of device **100** from inactive **101** to active **102.** The specified condition may be a dissolution of the dissolvable element(s), upon which the transition of treatment element **120** to operative state **122** occurs by a configurational change of medical device **100.** The dissolvable elements may comprise a band, a pin or a thread.

Holding element **130** may have a toroidal shape, e.g. be a ring or a band, flexible or stiff or made of surgical suture. Holding element **130** may be arranged to release parts of treatment element **120** by dissolution of dissolvable elements in holding element **130.** Holding element **130** may be made of plastic, metal, fabric, shape memory material and their combinations.

Holding element **130** may be associated with the activator that receives an external signal and operates treatment element **120** by cutting or dissolving holding element **130.**

For example (**Figure 1B**), holding element **130** may comprise a dissolvable ring or a set of dissolvable rings that are arranged to hold treatment element **120** in inoperative state **121,** and upon dissolution release treatment element **120** to take on operative state **122.** Treatment element **120** may comprise flaps connected to distal rim **119** and held against tubular adapter **110** either externally - embedded in tubular adapter **110** (left side of device **100** in **Figure 1B**) or external to tubular adapter **110** (right side of device **100** in Figure **1B**) by rings **130.**

The specified condition may comprise an external signal (e.g. electromagnetic radiation, ultrasound signal, magnetic or electric signal). Treatment element **120** may comprise an activator (not shown) arranged to receive the external signal and activate device **100.**

The specified condition may comprise an internal or an external stimulus of physical, mechanical, chemical electric or thermal nature. For example, the stimulus may comprise temperature, and the activation of device **100** may be carried out by drinking a hot fluid. In another example, activation of device **100** may be carried out by drinking a fluid having certain chemical character, such as reactivity with holding element **130,** induction of a certain pH level to dissolve holding element **130** etc.

The specified condition may comprise a pre-determined self activation, an exogenously induced activation (such as an external signal detected by an activator, or an induced activation by an administered substance causing e.g. a chemical reaction, a temperature change or an energy supply to device **100**) and an endoscopically performed activity (such as cutting holding element **130** to release treatment element **120**).

The specified condition may be incorporated into holding element **130** as a chemical or mechanical character that causes activation of device **100** after a specified period. In this case the specified condition is inherent in the structure or composition of holding element **130** or device **100.**

Dissolvable element as holding element **130** may be made of PLGA (poly (lactic-co-glycolic acid)), PGA (Polyglycolic acid), Caprolactones and any other known biocompatible and bio-dissolvable materials. The dissolution period of dissolvable element **130,** that may partly or fully determine the activation period of device **100,** may result from dissolution characteristics of the materials involved or from interaction of these materials with external signals, an internal or external stimulus, or intake liquids. For example, a patient may drink a liquid that promotes the dissolution of dissolvable elements **130,** like having chelators that scavenge divalent cations to break dissolvable elements **130,** or simply a hot fluid that promotes dissolution of dissolvable elements **130.** In embodiments, a mechanical intervention may be used to activate device **100,** for example cutting or releasing holding element **130** endoscopically.

To make it more flexible at insertion to stomach **85,** holding element **130** may be a flexible tape or yarn made of dissolvable materials. When these materials dissolve at the end of the healing period, e.g. in a week or more, device **100** changes to activated state **102,** e.g. taking on a final shape, and performs its therapeutic activity.

Treatment element **120** may comprise flaps that are positioned within tubular adapter **110.** Upon activation, device **100** may change it form to constrict the passage of food to the stomach.

Tubular adapter **110** may be made of at least one biocompatible semi-permeable flexible material (e.g. rubber, plastic, metal, carbon and other fibers, or any combination thereof) or non-permeable materials. The structure of tubular adapter **110** may be mesh-like, membranous, or fibrous at different part of adapter **110** or in adapter **110** as a whole. Tubular adapter **110** may have a longitudinally variable flexibility, selected to allow the connection of proximal rim **111** to inner lining **95,** to stabilize device **100,** to allow a change in a spatial configuration of device **100** upon its transition from inactive **101** to activated **102** state, and to provide a distal support of treatment element **120.**

In embodiments of device **100** as an anti-GERD device, such as a one way valve, adapter **110** and treatment element **120** may be made of impermeable material.

The mechanical strength of tubular adapter **110** may change longitudinally (e.g. **Figures 2A, 2B, 2C**) to generate a mechanical change of structure of device **100,** to perform the treatment (e.g. constriction of food passage). The flexibility and strength of tubular adapter **110** may change in different regions to support the transition from inactive state **101** to activated state **102** of device **100** and to support its functionality.

Tubular adapter **110** may have a longitudinally variable permeability selected to control fluid exchange between the stomach lumen and an internal volume of adapter **100.**

Tubular adapter **110** may comprise a rigid area **113** near proximal rim **111** that attenuates forces acting through tubular adapter **110** on attachment area **112.** Rigid area **113** may be ring shaped and arranged to protect attachment area **112** from forces that could be activated by tension in tubular adapter **110** in its activated state **102,** e.g. operating as an intake limiting pouch. Area **113** can be made rigid in tension but flexible enough to enable insertion of device **100** via the esophagus.

Treatment element **120** may comprise at least one supportive element **124** that is embedded within a more flexible distal part of tubular adapter **110** (**Figures 2A, 2B**). Supportive elements **124** may be rigid or flexible, and have varying thickness.

Supportive elements **124** may comprise rigid beams embedded longitudinally in tubular adapter **110,** or one or more rigid tubular elements. The rigid beams may be pivoted on at least one supportive element **126** embedded in tubular adapter **100** (**Figures 2A, 2B, 2C**) and are arranged, in operative state **122** of treatment element **120,** to distally constrict distal orifice **123** of device **100** upon proximal pressure applied by food internally on adapter **110.**

The distal ends of supportive elements **124** (e.g. rigid thin beams) may be interconnected by an annular element **147,** such as a thread yarn or an annular spring, arranged to define a size of distal orifice **123** and to allow an endoscopic manual manipulation of a size of distal orifice **123.** Distal orifice **123** may have a constant or an adjustable size. In particular, as illustrated below, a structure of the intake limiting pouch may be arranged to decrease a size of distal orifice **123** as a function of an increasing filled volume of the pouch, and vice versa. Either device **100** as a whole (adapter **110** and treatment element **120**) or treatment element **120** alone may function as the intake limiting pouch.

Annular element **147** may function as supportive element **126 -** to urge supportive elements **124** into place to change the spatial configuration of device **100,** e.g. to take the form of an intake limiting pouch in activated state **102.** Annular element **147** may have this function in device **100** either in a two part configuration (e.g. Figures **1B****,** **6**) or in one part configuration (**Figure 7**).

Supportive element **126** may comprise a rigid ring arranged to urge supportive elements **124** into place. Holding element **130** may be arranged to restrain supportive elements **124** (e.g. the rigid beams) in inactive state **101** of device **100,** and to release supportive elements **124** to deform device **100** to a form of the intake limiting pouch, or any other active form. Pivot **126** may comprise a rigid ring that urges the rigid beams to deform device **100** into the activated formation.

Supportive elements **124** may be embedded in tubular adapter **110** with a mechanical tendency to bend device **100** inwards, i.e. to change from inoperative state **121** (**Figure 2A**) to operative state **122** (**Figures 2B, 2C**). Supportive elements **124** may be pressed to retain inoperative state **121** by biodissolvable ring **130.**

Treatment element **120** may further comprise a pivot as supportive element **126** connected to supportive elements **124** and arrange to support a turning movement of supportive elements **124** in respect to pivot **126,** to generate a constriction of distal rim **119,** controlling the size of orifice **123.** Due to the constriction, device **100** takes, in activated state **102** a form of an intake limiting pouch.

Furthermore, the proximal end of at least one supportive element **124** may be effected by the content of tubular adapter **110.** For example, as the pouch formed in activated state **102** of device **100** gets fuller, supportive element **124** may increase their turning angle around pivot **126** and so decrease the size of orifice **123** to generate a larger resistance to food movement through device **100** and thereby a feeling of satiation. As a result, when ring **130** dissolves, device **100** turns into an intake limiting pouch with a self-regulating outlet **123.**

Outlet orifice **123** that limits the evacuation of food from the pouch into the stomach, may be of fixed size, or be self adjusting that such as to change its outlet area as a function of the fullness of the pouch, namely the fuller the pouch the smaller the exit area and vice-versa - when the pouch is empty the exit area may be larger, back to its original size.

Supportive elements **124** may have a variable elasticity, for example a higher elasticity on their distal end to allow a large range of sizes for orifice **123.** Distal end **119** of tubular adapter may have a corresponding elasticity.

In embodiments of device **100** as an intake limiting pouch with distal orifice **123,** the size of orifice **123** may be either fixed or decreasing by an increasing filled volume of the pouch. Orifice **123** may be adjustable and /or periodically openable according to specified criteria. At least one supportive element **124** may be flexible and comprise a thread yarn that may be tightened endoscopically if needed to regulate the size of orifice **123.** Treatment element **120** may comprise a thread yarn embedded around distal orifice **123** and arranged to allow an endoscopic manual manipulation of a size of distal orifice **123.** Treatment element **120** may comprise a distal circumferential spring, arranged to constrict distal orifice **123.**

In embodiments, tubular adapter **110** may maintain a cylindrical shape in both inactive **101** and activated **102** states of device **100,** while treatment element **120** forms alone the constriction of distal opening or orifice **123** of the formed intake limiting pouch.

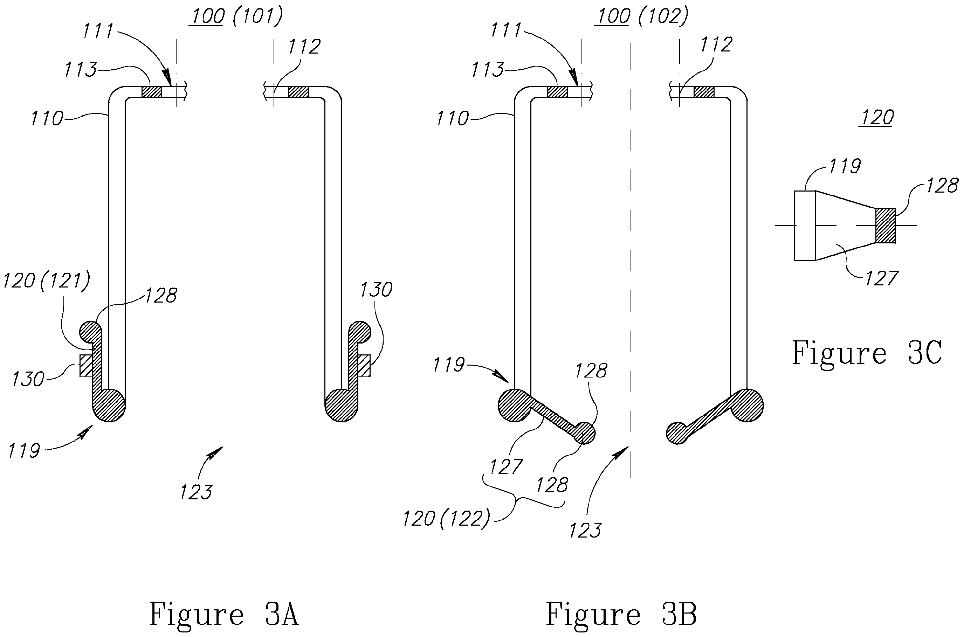

For example (**Figures 3A, 3B**), treatment element **120** may comprise flaps **127** arranged to be held by dissolvable ring **130** in inoperative state **121** and released in operative state **122,** with distal rim **119** serving as a pivot. Flaps **127** may be trapezoid (**Figures 3C**) and have rounded edges **128** that define orifice **123.** Flaps **127** may be held in place by the elasticity of distal rim **119** (distal rim **119** may be pre-tensioned) or by attached elements (not shown).

In embodiments, treatment element **120** may form a one way valve arranged to prevent stomach fluid from reaching esophagus **80** (**Figures 4A, 4B**). Flaps **127** may be triangular (**Figures 4C**) and may in operative state **122** close orifice **123** partially or completely. Flaps **127** may be held in activated state **102** of device **100** such as to allow only downwards movement (into stomach **85**) of food from esophagus **80** through device **100,** and prevent backward movement - up into device **100** and esophagus **80.** For example, flaps **127** may be stopped by detents **129** from folding away from closed orifice **123** upon upward pressure of stomach fluids. Flaps **127** may move downwards, against a spring force (e.g. exerted by distal rim **119** or an additional spring element) to let food enter stomach **85** smoothly, but flaps **127** may be disabled from moving upward beyond the fully closed shape when no pressure is activated from esophagus **80** into stomach **85.** Device **100** thus functions as an anti GERD (Gastroesophageal reflux disease) element that lets food exit from device **100** but does not let stomach juices or any other elements enter device **100.** Flaps **127** may be held in inoperative state **121** by holding elements **130.** In this case device **100** is made of non permeable materials.

**Figure 5** illustrates another embodiment of device **100** as an anti GERD element, an embodiment in which flaps **127** are folded internally within the distal end of tubular adapter **110.** Like the embodiment in **Figure 4B****,** detents **129** prevent flaps **127** from folding in the direction of esophagus **80,** thereby blocking stomach contact from entering device **100.** In the illustrated embodiment flaps **127** are trapezoidal, and close orifice **123** completely with some overlap that stabilizes the valve they create. The illustrated configuration comprises an attachment rim **131** that interlocks with distal rim **119** of tubular adapter **119** and affixes treatment element **120** thereto, as well as a pivot **132** for enabling movement of flaps **127** to allow food enter stomach **85.**

Treatment element **120** may be an intake limiting pouch (**Figure 6**) with distal orifice **123** having a size that is either fixed or decreasing by an increasing filled volume of the pouch. Treatment element **120** as the intake limiting pouch may have a sealable distal orifice **123** that is periodically opened according to specified criteria, to allow passage of food.

Treatment element **120** may be attached to tubular adapter **110** either during the insertion of tubular adapter **110** or later, e.g. after the healing has ended. The attachment of treatment element **120** as an intake limiting pouch to tubular adapter **110** may be carried out by rim **131** of treatment element **120** pressed into and held by flange **125** associated with distal rim **119** or by rim **119** itself. Rim **131** of treatment element **120** may be, for example, a toroidal flange, a bayonet, a screw types, a band type, or any flexible connection.

Treatment element **120** may comprise a distal circumferential spring (not shown), arranged to constrict distal orifice **123** of device **100.**

Medical device **100** may further comprise a drug delivering element **115** (**Figures 1C**, **1D**) arranged to support the healing and/or the treatment. Drug delivering element **115** may be arranged to deliver a drug into any of: stomach **85** lumen, stomach lining **95** (e.g. attachment area **112**), or a vicinity of esophagus **80.** For example, drug delivering element **115** may comprise a spring (**114**) loaded delivering element **116** arranged to pierce stomach lining **95** to deliver the drug. Delivering element **116** may have a sharp edge **106** surrounded by a stopping area **107** (**Figure 1E**) that prevents excessive penetration into stomach lining **95** as a result of peristaltic waves. Delivering element **116** may be pivoted at pivot **117** on a basal element **118.** Drug delivering element **115** may be restrained by holding element **130** and be released together with treatment element **120.** Alternatively, drug delivering element **115** may be restrained by an additional holding element **130,** and released before or after the release of treatment element **120.**

In embodiments (**Figures 4B****,** **5**), flaps **127** may be arranged to hold food or fluids within device **100** for a specified period before releasing it into stomach **85.** For this use flaps **127** may be reinforced and associated with a timing mechanism.

Treatment element **120** may be selected to be attached on a connected adapter **110** at any time after the specified period has passed. Moreover, treatment element **120** may be replaced by with another treatment element **120** (to apply a different or a modified treatment, or to renew an effective treatment element **120**) while maintaining adapter **110** connected to stomach **85.**

Inert tubular adapter **110** and treatment element **120** may be integrated, and device **100** may be functional in a single part configuration as an intake limiting pouch, as illustrated in **Figures 7** and **2C****.** Device **100** is connected proximally (**111**) at a connection area **112** to stomach **85** in around esophageal sphincter **90** (located below diaphragm **81**). Rim **119** of device **100** may comprise an outlet ring **142** that is toroidal and hollow.

One or more fluid containers **141** (**Figure 7**) may be attached to the inner lining of device **100** or be embedded in the wall of device **100.** Containers **141** may be connected to outlet ring **142** by one or more flow channels **143,** such as to fill hollow ring **142** under control of an inflating/deflating valve **144** connected to containers **141.** When the pouch fills with food, pressure is generated on one or more containers **141,** and the pressure passes into hollow ring **142** which inflates and reduces outlet exit **123.** When the food finally leaves the pouch, the pressure diminishes and the outlet area shrinks back to its initial size. The baseline size of outlet **123** may be determined by setting the initial pressure within device **100.** For example, valve **144,** that may be located within the pouch may be arranged to enable change of the initial pressure via endoscopic means. This embodiment may be implemented in device **100** either in its one part or in its two part configuration.

In another embodiment (**Figure 2C**), thin and narrow beams **124** are embedded within the pouch or attached to the pouch. When the pouch expands as it fills with food, beams **124** move or bend to the outside direction with the pouch body, but because the lower, distal part is kept in constant perimeter by a limiting structural element as supportive element **126,** that is connected distally to device **100** the outlet exit area **123** is reduced by the lower parts of thin beams **124.** Limiting structural element **126** may be enforced by reinforcement **147,** such as a cord, a spring, or other means. Other possibilities for implementing reinforcement **147** are mechanical hinges or shutters that can be moved to enable the insertion of device **100** via esophagus **80** (see for example **Figures 3A** and **3B**). Limiting structural element **126** or reinforcement **147** may comprise a round or flat metal spring attached to the pouch, embedded in it or threaded into the holes in bulges that are part of the pouch. The spring may be inserted into the stomach having a small diameter, and expanded to a larger diameter after insertion, and locked in the opened state. The larger diameter in the opened state may be selected to eliminate the possibility of a free pouch to pass via pylorus **96,** typically larger than 25 mm .The area of outlet **123** is regulated by pouch volume. A drain tube may be attached to the pouch, near outlet **123** and drain all or part of the food from pouch to pylorus **96** or to distal parts of stomach **85.** The tube can be an inherent part of the pouch, alternatively it can be attached permanently, or attached with a detachable connection. The attachment is flexible and does not hinder the variation of outlet area **123.** The tube may lead through stomach **85,** through pylorus **96** and down below the duodenum. In this embodiment the food passes through the drain tube directly to the small intestine.

**Figure 8** is a high level flowchart illustrating a gastric treatment method **150,** according to some embodiments of the invention.

Method **150** comprises the following stages: connecting an inert tubular adapter to an inner lining of the stomach (stage **155**) to encircle the esophageal sphincter, the connection characterized by a specified healing period, affixing a treatment element to the inert tubular adapter (stage **160**), and operating the treatment element to apply a treatment (stage **165**), wherein the operating (stage **165**) is carried out a specified period after the connecting (stage **155**), the specified period being equal to or longer than the healing period, to temporally separate the connection of the tubular adapter (stage **155**) and the application of the treatment (stage **165**).

Method **150** may comprise restricting a food intake (stage **185**), wherein the treatment is creating a partial or full enclosure of an adapter volume (stage **186**), and controllably releasing the food from the enclosure (stage **187**).

Method **150** may comprise preventing GERD (stage **190**), wherein the treatment is allowing a movement of fluids through the adapter in one direction only (stage **191**), namely from the esophagus to the stomach and not in the opposite direction (stage **192**).

Method **150** may further comprise delivering a drug (stage **195**) to the surroundings of the adapter.

Connecting (stage **155**) and affixing (stage **160**) may be carried out in a single procedure, and operating (stage **165**) may be carried out later, either by a natural (stage **170**) or artificial (stage **175**) dissolution of a dissolvable element that restrains the treatment element from operating when affixed to the adapter (stage **162**), or by an external signal (stage **180**). Alternatively, affixing (stage **160**) may be carried out in a separate procedure from connecting (stage **155**).

Affixing (stage **160**) may comprise at least partially embedding the treatment element within the inert tubular adapter (**163**), or pivoting the treatment element on a distal rim of the inert tubular adapter (**164**).

Operating (stage **165**) may comprise changing a spatial configuration of the adapter (stage **167**), e.g. to cause the adapter to take on a pouch form. Method **150** may further comprise controlling a size of a distal orifice of the pouch (stage **188**), externally or mechanically according to a degree of fullness of the pouch.

Method **150** may further comprise integrating the adapter and the treatment element to a single device (stage **199**).

Method **150** may further comprise selecting a treatment element (stage **157**), e.g. as an intake limiting pouch, an anti-GERD device, a drug delivering device etc. according to the clinical status of the patient. Method **150** may further comprise replacing one treatment element with another treatment element (stage **197**), while maintaining the adapter connected to the stomach.

In the above description, an embodiment is an example or implementation of the invention. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Furthermore, it is to be understood that the invention can be carried out or practiced in various ways and that the invention can be implemented in embodiments other than the ones outlined in the description above.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments.

## Claims

1. A medical device (100) comprising:
an inert tubular adapter (110) comprising a proximal rim (111) arranged to be connectable to an inner lining (95) of the stomach (85) to encircle the esophageal sphincter, and
a treatment element (120) affixed to the inert tubular adapter;
**characterized in that**:
the device has an active state (122) in which the treatment element is operative to constrict the passage of food to the stomach and an inactive state (121) in which the treatment element (120) is not operative, the treatment element having a delayed activation mechanism;
the tubular adapter (110) and the treatment element (120) are arranged to be endoscopically inserted into the stomach (85) by a single insertion procedure, with the treatment element (120) in the inactive state (121), and
the delayed activation mechanism is arranged to be mechanically or chemically self-activated or activated externally after a specified period following insertion.

2. The medical device of claim 1, wherein the delayed release mechanisim comprises an activator arranged to receive an external signal as the specified condition and to activate the treatment element thereupon, wherein the external signal is at least one of: an electrical signal, a magnetic signal and an acoustic signal.

3. The medical device of claim 1, further comprising at least one holding element arranged to restrain the treatment element (120) in the inactive state (121), and to release the treatment element (120), to the operative state (122) to activate the device (100).

4. The medical device of claim 3, wherein the at least one holding element (130) is toroidal, surrounds the adapter (110) and the treatment element (120), comprises at least one of: a ring; a band; and a thread, and is made of at least one of: plastic, metal, fabric and shape memory material.

5. The medical device of claim 3 or 4, wherein the at least one holding element (130) comprises at least one dissolvable element, wherein the self-activation is a dissolution of the at least one dissolvable element, upon which the transition of the treatment element (120) to the operative state (122) changes a spatial configuration of the medical device (100), wherein the at least one dissolvable element comprises at least one of: a ring; a band; a pin and a thread.

6. The medical device of claim 3, 4 or 5, wherein the at least one holding element (130) comprises at least one dissolvable element, wherein the self-activation is a dissolution of the at least one dissolvable element, upon which the transition of the treatment element (120) to the operative state (122) changes a spatial configuration of the medical device (100), wherein the at least one dissolvable element is at least one dissolvable ring or band or thread yarn, and wherein the treatment element is connected to the distal rim and held in its inactive state by the dissolvable ring or band or thread yarn.

7. The medical device of any one of claims 1-6, wherein the tubular adapter (110) has a longitudinally variable permeability selected to control fluid exchange between the stomach lumen and an internal volume of the adapter (110).

8. The medical device of claim 1, wherein at least one of: the treatment element (120), and the medical device (100) as a whole, is an intake limiting pouch with a distal orifice having an adjustable size.

9. The medical device of claim 8, wherein a structure of the intake limiting pouch is arranged to decrease a size of the distal orifice as a function of an increasing filled volume of the pouch, and vice versa.

10. The medical device of claim 8, wherein the treatment element (120) comprises a plurality of rigid beams that are pivoted on at least one supportive element (126) embedded in the tubular adapter (110) and are arranged, in the operative state of the treatment element (120), to constrict the distal orifice (123) as a function of a pressure applied by food internally on the adapter (110).

11. The medical device of claim 10, further comprising a holding element (130) arranged to restrain the rigid beams in the inactive state of the device (101), and to release the rigid beams to deform the device (100) to a form of the intake limiting pouch.

12. The medical device of claim 10, wherein the at least one supportive element (126) comprises an annular element (147) arranged to define a size of the distal orifice (123), wherein the annular element is further arranged to allow an endoscopic manual manipulation of the size of the distal orifice (123) and wherein the annular element (147) is a tied thread yarn or an annular spring.

13. The medical device of any one of claims 1-12, wherein the proximal rim is arranged to enable its connection to the inner lining of the stomach by at least one of: suturing, gluing, clipping, stapling, and riveting.

## Patentansprüche

1. Medizinische Vorrichtung (100), umfassend:
einen inerten röhrenförmigen Adapter (110), der einen proximalen Rand (111) umfasst, der angeordnet ist, um mit einer inneren Auskleidung (95) des Magens (85) verbindbar zu sein, um den Schließmuskel der Speiseröhre zu umgeben, und
ein Behandlungselement (120), das an dem inerten röhrenförmigen Adapter befestigt ist;
**dadurch gekennzeichnet, dass**:
die Vorrichtung einen aktiven Zustand (122) aufweist, in dem das Behandlungselement betriebsfähig ist, um den Durchgang von Nahrung in den Magen zu beschränken, und einen inaktiven Zustand (121), in dem das Behandlungselement (120) nicht betriebsfähig ist, wobei das Behandlungselement einen verzögerten Aktivierungsmechanismus aufweist;
der röhrenförmige Adapter (110) und das Behandlungselement (120) angeordnet sind, um durch ein einziges Einführungsverfahren endoskopisch in den Magen (85) eingeführt zu werden, wobei das Behandlungselement (120) sich im inaktiven Zustand (121) befindet, und
der verzögerte Aktivierungsmechanismus angeordnet ist, um mechanisch oder chemisch selbstaktiviert zu sein oder nach einer bestimmten Zeit nach dem Einsetzen extern aktiviert zu werden.

2. Medizinische Vorrichtung nach Anspruch 1, wobei der verzögerte Freisetzungsmechanismus einen Aktivator umfasst, der angeordnet ist, ein externes Signal als den angegebenen Zustand zu empfangen und daraufhin das Behandlungselement zu aktivieren, wobei das externe Signal mindestens eines der Folgenden ist: ein elektrisches Signal, ein magnetisches Signal und ein akustisches Signal.

3. Medizinische Vorrichtung nach Anspruch 1, weiter umfassend mindestens ein Halteelement, das angeordnet ist, um das Behandlungselement (120) in dem inaktiven Zustand (121) zu halten, und um das Behandlungselement (120) in den Betriebszustand (122) freizugeben, um die Vorrichtung (100) zu aktivieren.

4. Medizinische Vorrichtung nach Anspruch 3, wobei das mindestens eine Halteelement (130) ringförmig ist, den Adapter (110) und das Behandlungselement (120) umgibt, mindestens eines der Folgenden umfasst: einen Ring; ein Band; und ein Gewinde und aus mindestens einem der Folgenden besteht: Kunststoff, Metall, Stoff und Formgedächtnismaterial.

5. Medizinische Vorrichtung nach Anspruch 3 oder 4, wobei das mindestens eine Halteelement (130) mindestens ein lösliches Element umfasst, wobei die Selbstaktivierung eine Auflösung des mindestens einen löslichen Elements ist, worauf der Übergang des Behandlungselements (120) in den Betriebszustand (122) eine räumliche Konfiguration der medizinischen Vorrichtung (100) verändert, wobei das mindestens eine lösliche Element mindestens eines der Folgenden umfasst: einen Ring; ein Band; einen Stift und ein Gewinde.

6. Medizinische Vorrichtung nach Anspruch 3, 4 oder 5, wobei das mindestens eine Halteelement (130) mindestens ein lösliches Element umfasst, wobei die Selbstaktivierung eine Auflösung des mindestens einen löslichen Elements ist, worauf der Übergang des Behandlungselements (120) in den Betriebszustand (122) eine räumliche Konfiguration der medizinischen Vorrichtung (100) verändert, wobei das mindestens eine lösliche Element mindestens ein löslicher Ring oder ein lösliches Band oder ein lösliches Fadengarn ist, und wobei das Behandlungselement mit dem distalen Rand verbunden ist und durch den löslichen Ring oder das lösliche Band oder das lösliche Fadengarn in seinem inaktiven Zustand gehalten wird.

7. Medizinische Vorrichtung nach einem der Ansprüche 1-6, wobei der röhrenförmige Adapter (110) eine in Längsrichtung variable Permeabilität aufweist, die ausgewählt ist, um den Flüssigkeitsaustausch zwischen dem Lumen des Magens und einem Innenvolumen des Adapters (110) zu steuern.

8. Medizinische Vorrichtung nach Anspruch 1, wobei mindestens eines von: dem Behandlungselement (120) und der medizinischen Vorrichtung (100) als Ganzes ein Aufnahmebegrenzungsbeutel mit einer distalen Öffnung mit einer einstellbaren Größe ist.

9. Medizinische Vorrichtung nach Anspruch 8, wobei eine Struktur des Aufnahmebegrenzungsbeutels angeordnet ist, um eine Größe der distalen Öffnung als Funktion eines immer mehr zunehmenden Volumens des Beutels zu reduzieren und umgekehrt.

10. Medizinische Vorrichtung nach Anspruch 8, wobei das Behandlungselement (120) eine Vielzahl starrer Balken umfasst, die an mindestens einem Stützelement (126) angelenkt sind, das in dem röhrenförmigen Adapter (110) eingebettet ist und im Betriebszustand des Behandlungselements (120) angeordnet sind, um die distale Öffnung (123) in Abhängigkeit eines Drucks zu verengen, der durch Nahrung intern auf den Adapter (110) aufgebracht wird.

11. Medizinische Vorrichtung nach Anspruch 10, weiter umfassend eine Halteelement (130), das angeordnet ist, um die starren Balken im inaktiven Zustand der Vorrichtung (101) zu halten und die starren Balken freizugeben, um die Vorrichtung (100) zu verformen, um den Aufnahmebegrenzungsbeutel zu bilden.

12. Medizinische Vorrichtung nach Anspruch 10, wobei das mindestens eine Stützelement (126) ein ringförmiges Element (147) umfasst, das angeordnet ist, um eine Größe der distalen Öffnung (123) zu definieren, wobei das ringförmige Element weiter angeordnet ist, um eine endoskopische manuelle Manipulation der Größe der distalen Öffnung (123) zuzulassen und wobei das ringförmige Element (147) ein angebundenes Fadengarn oder eine Ringfeder ist.

13. Medizinische Vorrichtung nach einem der Ansprüche 1-12, wobei der proximale Rand angeordnet, um seine Verbindung mit der inneren Auskleidung des Magens durch mindestens eines von Nähen, Kleben, Beschneiden, Heften und Nieten zu ermöglichen.

## Revendications

1. Dispositif médical (100) comprenant :
un adaptateur tubulaire inerte (110) comprenant un rebord proximal (111) agencé pour pouvoir être relié à un revêtement interne (95) de l'estomac (85) pour encercler le sphincter oesophagien, et
un élément de traitement (120) fixé à l'adaptateur tubulaire inerte ;
**caractérisé en ce que** :
le dispositif possède un état actif (122) dans lequel l'élément de traitement est opérationnel pour resserrer le passage d'aliments vers l'estomac et un état inactif (121) dans lequel l'élément de traitement (120) n'est pas opérationnel, l'élément de traitement comportant un mécanisme d'activation retardée ;
l'adaptateur tubulaire (110) et l'élément de traitement (120) sont agencés pour être insérés par endoscopie dans l'estomac (85) par une procédure unique d'insertion, avec l'élément de traitement (120) à l'état inactif (121), et
le mécanisme d'activation retardée est agencé pour être auto-activé mécaniquement ou chimiquement ou activé de l'extérieur après une période spécifiée après l'insertion.

2. Dispositif médical selon la revendication 1, dans lequel le mécanisme de libération retardée comprend un activateur agencé pour recevoir un signal externe sous une condition spécifiée, et pour activer l'élément de traitement à sa réception, dans lequel ledit signal externe est au moins l'un parmi : un signal électrique, un signal magnétique et un signal acoustique.

3. Dispositif médical selon la revendication 1, comprenant en outre au moins un élément de retenue agencé pour retenir l'élément de traitement (120) à l'état inactif (121), et pour libérer l'élément de traitement (120) à l'état opérationnel (122) pour activer le dispositif (100).

4. Dispositif médical selon la revendication 3, dans lequel ledit au moins un élément de retenue (130) est toroïdal, entoure l'adaptateur (110) et l'élément de traitement (120), comprend au moins l'un parmi : un anneau ; une bande ; et un fil, et est constitué d'au moins l'un parmi : du plastique, du métal, du tissu et un matériau à mémoire de forme.

5. Dispositif médical selon la revendication 3 ou 4, dans lequel ledit au moins un élément de retenue (130) comprend au moins un élément soluble, dans lequel l'auto-activation est une dissolution dudit au moins un élément soluble, lors de laquelle le passage de l'élément de traitement (120) à l'état opérationnel (122) change une configuration spatiale du dispositif médical (100), dans lequel ledit au moins un élément soluble comprend au moins l'un parmi : un anneau ; une bande ; une épingle et un fil.

6. Dispositif médical selon la revendication 3, 4 ou 5, dans lequel ledit au moins un élément de retenue (130) comprend au moins un élément soluble, dans lequel l'auto-activation est une dissolution dudit au moins un élément soluble, lors de laquelle le passage de l'élément de traitement (120) à l'état opérationnel (122) change une configuration spatiale du dispositif médical (100), dans lequel ledit au moins un élément soluble est au moins un anneau ou bande ou fil filé soluble, et dans lequel l'élément de traitement est relié au rebord distal et maintenu dans son état inactif par l'anneau ou bande ou fil filé soluble.

7. Dispositif médical selon l'une quelconque des revendications 1 à 6, dans lequel l'adaptateur tubulaire (110) a une perméabilité longitudinalement variable, sélectionnée pour réguler un échange de fluide entre la lumière stomacale et un volume interne de l'adaptateur (110).

8. Dispositif médical selon la revendication 1, dans lequel au moins l'un parmi : l'élément de traitement (120) et le dispositif médical (100) dans son ensemble, est une poche de limitation d'ingestion avec un orifice distal présentant une taille réglable.

9. Dispositif médical selon la revendication 8, dans lequel une structure de la poche de limitation d'ingestion est agencée pour diminuer une taille de l'orifice distal en fonction d'un volume de remplissage croissant de la poche, et inversement.

10. Dispositif médical selon la revendication 8, dans lequel l'élément de traitement (120) comprend une pluralité de poutres rigides qui sont pivotées sur au moins un élément de soutien (126) intégré dans l'adaptateur tubulaire (110) et sont agencées, à l'état opérationnel de l'élément de traitement (120), pour resserrer l'orifice distal (123) en fonction d'une pression appliquée intérieurement par les aliments sur l'adaptateur (110).

11. Dispositif médical selon la revendication 10, comprenant en outre un élément de retenue (130) agencé pour retenir les poutres rigides à l'état inactif du dispositif (101), et pour libérer les poutres rigides afin de déformer le dispositif (100) en une forme de la poche de limitation d'ingestion.

12. Dispositif médical selon la revendication 10, dans lequel ledit au moins un élément de soutien (126) comprend un élément annulaire (147) agencé pour définir une taille de l'orifice distal (123), dans lequel l'élément annulaire est en outre agencé pour permettre une manipulation manuelle endoscopique de la taille de l'orifice distal (123) et dans lequel l'élément annulaire (147) est un fil filé noué ou un ressort annulaire.

13. Dispositif médical selon l'une quelconque des revendications 1 à 12, dans lequel le rebord proximal est agencé pour permettre sa liaison au revêtement interne de l'estomac par au moins l'un parmi : une suture, un collage, un clipsage, un agrafage et un rivetage.
